# EUROPEAN PATENT SPECIFICATION

(11) **EP 2 603 248 B1**
(45) Date of publication and mention of the grant of the patent: **03.01.2018**
(21) Application number: 11748831.2
(22) Date of filing: 09.08.2011
(51) Int. Cl.: A61L 27/48, A61L 27/50

(54) **REGENERATIVE TISSUE SCAFFOLDS**
REGENERATIVE GEWEBEGERÜSTE
ECHAFAUDAGES TISSULAIRES RÉGÉNÉRATEURS

(30) Priority: 10.08.2010 US 372339 P
(43) Date of publication of application: 19.06.2013
(73) Proprietor: Lifecell Corporation, Branchburg, NJ 08876-3876 (US)
(72) Inventor: OWENS, Rick, T., Stewartsville, New Jersey 08886 (US); ELMO, Laura, Clinton, New Jersey 08809 (US); LIU, Mike, Hillsborough, New Jersey 08844 (US); MAO, Yong, Basking Ridge, New Jersey 07920 (US)
(74) Representative: Ashton, Timothy
(86) International application number: PCT/US2011/047041
(87) International publication number: WO 2012/021490

(56) References cited:
- WO-A2-03/017826
- WO-A2-2007/134134
- LEE S J ET AL: "In vitro evaluation of a poly(lactide-co-glycolide)-collagen composite scaffold for bone regeneration", BIOMATERIALS, ELSEVIER SCIENCE PUBLISHERS BV., BARKING, GB, vol. 27, no. 18, 1 June 2006 (2006-06-01), pages 3466-3472, XP025097218, ISSN: 0142-9612, DOI: 10.1016/J.BIOMATERIALS.2006.01.059 [retrieved on 2006-06-01]

## Description

The invention is defined in the claims. The present disclosure relates to methods, mixtures, and kits for treating tissue or organ defects or injuries, including methods, mixtures, and kits for producing tissue scaffolds for treating tissue defects.

Human, animal, and synthetic materials are currently used in medical and surgical procedures to augment tissue or correct tissue defects. For certain purposes, materials with stable shapes are needed. In some cases, the manner of delivery of such material, e.g., by surgical procedure or by injection, may be important. Additionally, the ability of the material to not migrate away from the location in need of treatment may be needed.

Various current devices and methods for treating tissue or organ defects have had certain disadvantages. Accordingly, there is a need for improved devices and methods for treating tissue or organ defects. WO 2007/134134 discloses a tissue matrix for tissue repair. WO 2003/017826 discloses a biomaterial composition comprising a combination of processed tissue and a carrier. Lee *et al* discloses a hybrid composite scaffold composed of poly(d,l-lactide-co-glycolide) (PLGA) and a naturally derived collagen matrix for use in bone tissue regeneration.

Certain embodiments include a method (e.g., an ex vivo and/or an in vivo method) comprising providing a particulate acellular tissue matrix (ATM) and a solution comprising a polymer dissolved in a solvent. The method may further include mixing the solution with the particulate ATM to create a mixture, and placing the mixture in contact with an aqueous media. The solvent may diffuse from the mixture and a tissue scaffold may form.

Some embodiments include a tissue scaffold mixture comprising a particulate acellular tissue matrix (ATM), a polymer, and a water miscible solvent. When the mixture is placed in contact with an aqueous media, the water miscible
solvent may be capable of diffusing from the mixture to form tissue scaffold from the polymer and ATM.

### Brief Description of Drawings

Figure 1 is a flow chart showing a process for preparing a tissue scaffold.
Figure 2 illustrates implantation of a tissue scaffold in a defect, according to certain embodiments.
Figure 3A is a hematoxylin and eosin-stained four-week sub-dermal explant comprising PCL under 400x magnification, as described in Example 1.
Figure 3B is a hematoxylin and eosin-stained four-week sub-dermal explant from an injectable tissue scaffold comprising PCL, pADM, and dioxane under 400x magnification according to a process described in Example 1.
Figure 3C is a hematoxylin and eosin-stained four-week sub-dermal explant from an injectable tissue scaffold comprising PCL, pADM, and NMP under 400x magnification according to a process described in Example 1.
Figure 3D is a hematoxylin and eosin-stained four-week sub-dermal explant comprising P4HB under 400x magnification, as described in Example 1.
Figure 3E is a hematoxylin and eosin-stained four-week sub-dermal explant from an injectable tissue scaffold comprising P4HB, pADM, and dioxane under 400x magnification according to a process described in Example 1.
Figure 3F is a hematoxylin and eosin-stained four-week sub-dermal explant from an injectable tissue scaffold comprising P4HB, pADM, and NMP under 400x magnification according to a process described in Example 1.
Figure 4A is a hematoxylin and eosin-stained twelve-week sub-dermal explant comprising PCL under 400x magnification, as described in Example 1.
Figure 4B is a hematoxylin and eosin-stained twelve-week sub-dermal explant from an injectable tissue scaffold comprising PCL, pADM, and dioxane under 400x magnification according to a process described in Example 1.
Figure 4C is a hematoxylin and eosin-stained twelve-week sub-dermal explant from an injectable tissue scaffold comprising PCL, pADM, and NMP under 400x magnification according to a process described in Example 1.
Figure 4D is a hematoxylin and eosin-stained twelve-week sub-dermal explant comprising P4HB under 400x magnification, as described in Example 1.
Figure 4E is a hematoxylin and eosin-stained twelve-week sub-dermal explant from an injectable tissue scaffold comprising P4HB, pADM, and dioxane under 400x magnification according to a process described in Example 1.
Figure 4F is a hematoxylin and eosin-stained twelve-week sub-dermal explant from an injectable tissue scaffold comprising P4HB, pADM, and NMP under 400x magnification according to a process described in Example 1.
Figure 5A is a hematoxylin and eosin-stained four-week femoral condyle explant from an injectable tissue scaffold comprising BHA, pADM, and NMP under 20x magnification according to a process described in Example 2.
Figure 5B is a hematoxylin and eosin-stained four-week femoral condyle explant from an injectable tissue scaffold comprising BHA, pADM, and NMP under 100x magnification according to a process described in Example 2.
Figure 5C is a hematoxylin and eosin-stained four-week femoral condyle explant from an injectable tissue scaffold comprising BHA, pADM, and NMP under 400x magnification according to a process described in Example 2.
Figure 6 is an image of a femoral condyle twelve weeks after an injectable tissue scaffold comprising BHA, pADM, and NMP was implanted into a tissue defect.

### Description of Exemplary Embodiments

The invention is defined in the claims. In this application, the use of the singular includes the plural unless specifically stated otherwise. In this application, the use of "or" means "and/or" unless stated otherwise. Furthermore, the use of the term "including," as well as other forms, such as "includes" and "included," is not limiting.

The section headings used herein are for organizational purposes only and are not to be construed as limiting the subject matter described.

It will be understood that the benefits and advantages described herein may relate to one embodiment or may relate to several embodiments. It will further be understood that reference to 'an' item refers to one or more of those items.

The steps of the methods described herein may be carried out in any suitable order, or simultaneously where appropriate.

Where appropriate, aspects of any of the examples described herein may be combined with aspects of any of the other examples described to form further examples having comparable or different properties and addressing the same or different problems.

It will be understood that the description herein of preferred embodiments is given by way of example only and that various modifications may be made by those skilled in the art. The specification, examples, and data herein provide a complete description of the structure and use of exemplary embodiments of the invention.

The term "acellular tissue matrix" (ATM), as used herein, refers generally to any tissue matrix that is substantially free of cells and/or cellular components. Skin, parts of skin (e.g., dermis), and other tissues, such as blood vessels, heart valves, fascia, cartilage, bone, nerve, and connective tissues may be used to create acellular matrices within the scope of the present disclosure (e.g., by the removal of the cells and/or cellular components). Acellular tissue matrices can be tested or evaluated to determine if they are substantially free of cell and/or cellular components in a number of ways. For example, processed tissues can be inspected with light microscopy to determine if cells (live or dead) and/or cellular components remain. In addition, certain assays can be used to identify the presence of cells or cellular components. For example, DNA or other nucleic acid assays can be used to quantify remaining nuclear materials within the tissue matrices. Generally, the absence of remaining DNA or other nucleic acids will be indicative of complete decellularization (i.e., removal of cells and/or cellular
components). Finally, other assays that identify cell-specific components (e.g., surface antigens) can be used to determine if the tissue matrices are acellular.

The tissue scaffolds of the present disclosure can include an ATM that has the biologic ability to support tissue regeneration. In some embodiments, tissue scaffolds can support cell ingrowth and differentiation. For example, the scaffolds can be used for tissue ingrowth, orthopedic surgery, periodontal applications, tissue remodeling, or tissue restoration. In one embodiment, the tissue scaffolds produce a regenerative tissue response, as demonstrated by the presence of fibroblast-like cells and blood vessels.

In various embodiments, the tissue scaffolds can be used for treatment of numerous different anatomical sites and can be used in a wide array of applications. Certain exemplary applications include, but are not limited to, absorptive dressings, dermal regeneration (e.g., for treatments of all types of ulcers and burns), nerve regeneration, cartilage regeneration, connective tissue regeneration or repair, bone regeneration, wound/foam lining, integrated bandage dressings, substrate/base for skin grafts, vascular regeneration, cosmetic surgery, metal and/or polymer implant coating (for example, to increase implant integration and biocompatibility), and replacement of lost tissue (e.g., after trauma, breast reduction, mastectomy, lumpectomy, parotidectomy, or excision of tumors).

The tissue scaffolds can elicit a reduced immunological or inflammatory response when implanted in an animal compared to the polymer or polymers used to produce the scaffold alone. The effect of the tissue scaffold in the host can be tested using a number of methods. For example, in some embodiments, the effect of the tissue scaffold in the host can be tested by measuring immunological or inflammatory response to the implanted scaffold. The immunological or inflammatory response to the tissue scaffold can be measured by a number of methods, including histological methods. For example, explanted scaffold can be stained and observed under a microscope for histological evaluation, as described further below. In some embodiments, the immunological or inflammatory response to the scaffold can be demonstrated by measuring the number of inflammatory cells (e.g., leukocytes). The attenuated immunological or inflammatory response to the scaffold may be associated with a reduced number of inflammatory cells, as described further below. For example, inflammatory cells can be measured through immuno-histochemical staining methods designed to identify lymphocytes, macrophages, and neutrophils. Immuno-histochemical methods may also be used to determine the presence of inflammatory cytokines including interleulin-1, TNF-alpha, and TGF-beta.

In various embodiments, tissue scaffolds of the present disclosure can be used to treat any of a wide range of disorders. Tissue defects can arise from many causes, including, for example, congenital malformations, traumatic injuries, infections, and oncologic resections. The tissue scaffolds can be used to treat musculoskeletal defects, e.g., as an articular graft to support cartilage regeneration. The tissue scaffolds can also be used to treat defects in any soft tissue, e.g., tissues that connect, support, or surround other structures and organs of the body. Soft tissue can be any non-osseous tissue.

The tissue scaffolds can be used to treat soft tissues in many different organ systems. These organ systems can include, but are not limited to, the muscular system, the genitourinary system, the gastroenterological system, the integumentary system, the circulatory system, and the respiratory system. The tissue scaffolds can also be useful to treat connective tissue, including the fascia, a specialized layer that surrounds muscles, bones, and joints of the chest and abdominal wall, and for repair and reinforcement of tissue weaknesses in urological, gynecological, and gastroenterological anatomy. In some embodiments, the tissue or organ in need of treatment can be selected from the group consisting of skin, bone, cartilage, meniscus, dermis, myocardium, periosteum, artery, vein, stomach, small intestine, large intestine, diaphragm, tendon, ligament, neural tissue, striated muscle, smooth muscle, bladder, urethra, ureter, and gingival.

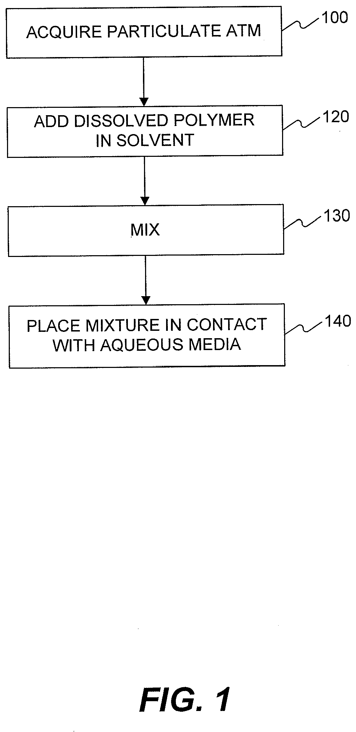

Figure 1 illustrates steps for preparing a tissue scaffold. The scaffolds can include a particulate ATM (step 100). In some embodiments the ATM can be derived from, for example, dermis, cartilage, bone, demineralized bone, blood vessels, heart valves, fascia, or nerve connective tissue. Preparation of particulate ATM is described in greater detail below. In some embodiments, the particulate ATM comprises particles of uniform size. The particulate ATM may comprise a dermal ATM. In some embodiments, the dermal ATM is a human tissue matrix. In some embodiments, the dermal ATM is a porcine tissue matrix. In some embodiments, the particulate ATM is a cartilage tissue matrix, which may be derived from human cartilage. In some embodiments, the cartilage tissue matrix is derived from porcine cartilage. In some embodiments, the particulate ATM comprises a bone tissue matrix. In some embodiments, the bone tissue matrix is derived from human bone. In some embodiments, the bone tissue matrix is derived from porcine bone.

The ATM can be selected to provide a variety of different biologic and mechanical properties. For example, the ATM can be selected to allow cell ingrowth and remodeling to allow regeneration of tissue normally found at the site where the matrix is implanted. For example, the ATM, when implanted on or into cartilage, may be selected to allow regeneration of the cartilage without excessive fibrosis or scar formation. In addition, the ATM may be selected to limit excessive inflammatory reaction and to produce tissue similar to the original host tissue. In some embodiments, the ATM comprises collagen, elastin, and vascular channels. Examples of ATMs are discussed further below.

In addition, the tissue scaffolds can include one or more polymeric materials, which can be selected from a number of polymer types. As used herein, the polymeric materials can include synthetic polymers and/or naturally occurring polymers. Furthermore, the polymeric materials can include individual polymers and/or polymer mixtures (copolymers). In some embodiments, the polymeric materials can include polyglycolide, polylactide, polydioxane (or other polyether esters), poly(lactide-co-glycolide), and/or polyhydroxyalkonates. For example, in certain embodiments, the polymeric material can include polyhydroxyalkonates such as, for example, polyhydroxybutyrate (e.g., poly-3-hydroxybutyrate, poly-4-hydroxybutyrate (P4HB)), polyhydroxyvalerate, polyhydroxyhexanoate, polyhydroxyoctanoate, or trimethylene carbonate. Alternatively or additionally, the polymeric material can include polycaprolactone (PCL) and/or hyaluronic acid derivatives (e.g., esters, anhydrides, etc.), such as, for example, a benzyl ester derivative of hyaluronic acid (BHA). In certain embodiments, the polymeric materials in the tissue scaffolds can provide a structure for the ATM. The structure can increase implant integration, biocompatibility, and stability and may prevent migration of the implant away from the treatment site. In addition, the inclusion of the ATM with the polymer in the tissue scaffolds can increase the acceptance of the polymer via attenuation or reduction of immunological or inflammatory response, as compared to an implant comprising only the polymer.

In some embodiments, the polymer can be dissolved in a suitable solvent (step 120) to form a polymer solution. As used herein, the solvent can include solvent mixtures. In certain embodiments, the solvent may be chosen based on the polymer being used and/or the environment in which it will be mixed or delivered from in order to be appropriately reactive so as to avoid undesired reactions. For example, in some embodiments, any solvent in which the particular polymer is soluble may be used. In certain embodiments, the solvent may be selected to be biocompatible and water miscible. The solvent selected may also be weakly volatile. In some embodiments, the solvent may include, for example, dioxane, N-methyl-2-pyrrolidone (NMP), and/or dimethyl sulfoxide (DMSO). Dissolving the polymer in the solvent may provide a solution of appropriate viscosity to accommodate thorough mixing with the particulate ATM and facilitate implantation of the combination (e.g., by injection, packing into a site, etc.). The polymer concentration may be manipulated to create a more or less viscous mixture.

As an example, in some embodiments, PCL may be dissolved in dioxane and/or NMP. In certain embodiments, the PCL dissolved in dioxane and/or NMP can be about 5-30% (w/v). In a further embodiment, the PCL dissolved in dioxane and/or NMP solvent can be 5%, 8%, 10%, 12%, 15%, 18%, 20%, 25%, or 30% (w/v); 5% to 30% (w/v); or 10% to 20% (w/v) and any values in between.

In other embodiments, P4HB can be dissolved in dioxane and/or NMP. In some embodiments, the P4HB dissolved in dioxane and/or NMP can be about 5-40% (w/v). In a further embodiment, the P4HB dissolved in dioxane and/or NMP can be 5%, 8%, 10%, 12%, 15%, 18%, 20%, 25%, 30%, or 40% (w/v); 5% to 40% (w/v); or 10% to 30% (w/v) and any values in between.

In other embodiments, BHA may be dissolved in DMSO and/or NMP. In some embodiments, the BHA dissolved in DMSO and/or NMP can be about 5-50% (w/v). In a further embodiment, the BHA dissolved in DMSO and/or NMP can be 5%, 8%, 10%, 12%, 15%, 18%, 20%, 25%, 30%, 40%, or 50% (w/v); 5% to 50% (w/v); 5% to 40% (w/v); 10% to 40% (w/v); or 10% to 30% (w/v) and any values in between.

Each of these scaffold materials may impart different properties upon the final product allowing for manipulation of *in vivo* turnover/persistence, biomechanical properties, and overall biological response.

The polymer solution can then be mixed with the particulate ATM (step 130). In some embodiments, the volume of polymer solution may be selected to provide a final concentration of 25% (w/w) of polymer overall when combined with the particulate ATM. The method for mixing the solution with the particulate ATM may be selected based on the intended location for forming the tissue scaffold. For example, in some embodiments, the solution may be mixed with the particulate ATM in any suitable receptacle. In other embodiments, the solution and particulate ATM may be mixed in one or more syringes. For example, the particulate ATM may be placed in a first syringe. A desired volume of polymer solution may be drawn into a second syringe. The first and second syringes may then be coupled, and the materials in each may be mixed by passing the materials between the syringes. The resulting final mixture may then be transferred to a single syringe. A needle or cannula may then be attached to the syringe to facilitate injection of the mixture

In one embodiment, some or all of the components of the tissue scaffold mixture may be premixed or prepackaged together. For example, in some embodiments, a polymer that is already dissolved in a solvent at a desired concentration may be provided for preparing the tissue scaffold mixture. Similarly, in some embodiments, a solution having a polymer dissolved in a solvent may be premixed with a particulate ATM, packaged in desired amounts, and stored for later use. Thus, the final tissue scaffold mixture may be prepared in advance of forming the tissue scaffold, and/or before storage, shipment, or sale.

The final mixture of particulate ATM, polymer, and solvent may then be placed in contact with an aqueous media (step 140), allowing the solvent to diffuse from the mixture to form a tissue scaffold from the polymer and ATM. In some *in vivo* embodiments, the final mixture may be placed in, on, or proximate to a tissue site. As discussed above, tissue scaffolds can be used in an array of applications and for treatment of many different anatomical sites. For example, in some embodiments, the final mixture may be placed into a tissue defect in soft tissue. Upon placement of the mixture in situ, the solvent may diffuse into the surrounding tissue or interstitial space.

In *ex vivo* embodiments, the final mixture may be placed in contact with an aqueous media prior to implantation. For example, in some embodiments, the final mixture may be placed in a mold having a desired shape. A mold may include an eppendorf tube, a metal tube, an injection tube, or a mold in the form of a tissue or organ defect into which the tissue scaffold will be implanted. In such embodiments, the final mixture and/or the mold may be exposed to an aqueous media to facilitate diffusion of the solvent from the mixture. For example, the final mixture and/or the mold may be rinsed, washed, and/or soaked in a bath to remove any or all of the solvent in the final mixture.

Diffusion of the solvent may leave a consistently distributed tissue scaffold of ATM and polymer. The resulting tissue scaffold may consist of regenerative tissue particles encased in a polymeric/synthetic support scaffold. In some embodiments, the tissue scaffold may have a three-dimensional shape that is stable under mechanical stress. As discussed above, the scaffold materials may be selected to achieve a tissue scaffold having particular biomechanical properties. Thus, depending on the intended use, the tissue scaffold may be rigid, elastic, resilient, and/or viscoelastic. In some embodiments, the scaffold materials may be selected to form a tissue scaffold having a stiffness that is substantially similar to that of the tissue at the target location. Further, in some embodiments, the tissue scaffold may also resist migration from the target location.

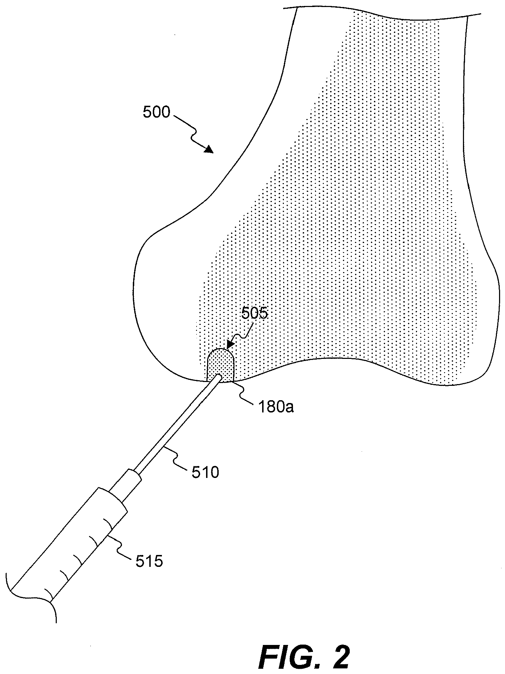

Figure 2 provides an exemplary illustration of implantation of a tissue scaffold to treat a defect 505 in a long bone 500 (e.g., femur or humerus). In various embodiments, a scaffold 180a can be implanted into the location of the defect 505. In some embodiments, the tissue scaffold 180a can be implanted by injection through a needle or cannula 510 coupled to a syringe 515 providing the tissue scaffold material.

### Acellular Tissue Matrices

The term "acellular tissue matrix" (ATM), as used herein, refers generally to any tissue matrix that is substantially free of cells and/or cellular components. Skin, parts of skin (e.g., dermis), and other tissues such as blood vessels, heart valves, fascia, cartilage, bone, and nerve connective tissue may be used to create acellular matrices within the scope of the present disclosure. Acellular tissue matrices can be tested or evaluated to determine if they are substantially free of cell and/or cellular components in a number of ways. For example, processed tissues can be inspected with light microscopy to determine if cells (live or dead) and/or cellular components remain. In addition, certain assays can be used to identify the presence of cells or cellular components. For example, DNA or other nucleic acid assays can be used to quantify remaining nuclear materials within the tissue matrices. Generally, the absence of remaining DNA or other nucleic acids will be indicative of complete decellularization (i.e., removal of cells and/or cellular components). Finally, other assays that identify cell-specific components (e.g., surface antigens) can be used to determine if the tissue matrices are acellular. Skin, parts of skin (e.g., dermis), and other tissues such as blood vessels, heart valves, fascia, cartilage, bone, and nerve connective tissue may be used to create acellular matrices within the scope of the present disclosure.

In general, the steps involved in the production of an ATM include harvesting the tissue from a donor (e.g., a human cadaver or animal source) and cell removal under conditions that preserve biological and structural function. For example, desired biologic and structural functions include the ability to support cell ingrowth and tissue regeneration, to provide mechanical support (e.g., to a surgical site or defect), and/or to prevent excessive immunologic response, inflammation, fibrosis, and/or scarring. In certain embodiments, the process includes chemical treatment to stabilize the tissue and avoid biochemical and structural degradation together with or before cell removal. In various embodiments, the stabilizing solution arrests and prevents osmotic, hypoxic, autolytic, and proteolytic degradation, protects against microbial contamination, and reduces mechanical damage that can occur with tissues that contain, for example, smooth muscle components (e.g., blood vessels). The stabilizing solution may contain an appropriate buffer, one or more antioxidants, one or more oncotic agents, one or more antibiotics, one or more protease inhibitors, and/or one or more smooth muscle relaxants.

The tissue is then placed in a decellularization solution to remove viable cells (e.g., epithelial cells, endothelial cells, smooth muscle cells, and fibroblasts) from the structural matrix without damaging the biological and structural integrity of the ATM (e.g., collagen matrix). The integrity of the ATM can be tested in a number of ways. For example, differential scanning calorimetry can be used to identify changes in thermal transition temperature that indicate cross-linking (elevation in transition temperature) or collagen degradation (decrease in transition temperature). In addition, electron microscopy can demonstrate changes in normal collagen patterns, and enzymatic digestion assays can demonstrate collagen damage. Further, the loss of various glycosaminoglycans (e.g., chondroitin sulfate and hyaluronic acid) can indicate an undesirable change in the tissue matrix.

The decellularization solution may contain an appropriate buffer, salt, an antibiotic, one or more detergents (e.g., TRITON X-100™, sodium deoxycholate, polyoxyethylene (20) sorbitan mono-oleate), one or more agents to prevent cross-linking, one or more protease inhibitors, and/or one or more enzymes. Suitable methods for producing ATM are described in, for example, H. Xu et al., A Porcine-Derived Acellular Dermal Scaffold That Supports Soft Tissue Regeneration: Removal of Terminal Galactose-α-(1,3)-Galactose and Retention of Matrix Structure. Tissue Eng. Part A, Vol. 15, 1-13 (2009) ("Xu"). In particular, the paragraph under the subheading "Test materials" on page 2 of Xu describes a suitable method for producing ATM from porcine skin.

After the decellularization process, the tissue sample is washed thoroughly with saline. In some exemplary embodiments, e.g., when xenogenic material is used, the decellularized tissue is then treated overnight at room temperature with a deoxyribonuclease (DNase) solution. In some embodiments, the tissue sample is treated with a DNase solution prepared in DNase buffer (20 mM HEPES (4-(2-hydroxyethyl)-1-piperazineethanesulfonic acid), 20 mM CaCl₂ and 20 mM MgCl₂). Optionally, an antibiotic solution (e.g., Gentamicin) may be added to the DNase solution. Any suitable buffer can be used as long as the buffer provides suitable DNase activity.

While an ATM may be made from one or more individuals of the same species as the recipient of the tissue scaffold, this is not necessarily the case. Thus, for example, an ATM in the tissue scaffold may be made from porcine tissue. Species that can serve as recipients of ATM and donors of tissues or organs for the production of the ATM include, without limitation, mammals, such as humans, nonhuman primates (e.g., monkeys, baboons, or chimpanzees), pigs, cows, horses, goats, sheep, dogs, cats, rabbits, guinea pigs, gerbils, hamsters, rats, or mice.

Elimination of the Galα1-3Galβ1-(3)4GlcNAc-R epitopes ("α-gal epitopes") from the ATM may diminish the immune response against the ATM. The α-gal epitope is expressed in non-primate mammals and in New World monkeys (monkeys of South America) on macromolecules such as glycoproteins of the extracellular components. U. Galili et al., J. Biol. Chem. 263: 17755 (1988). This epitope is absent in Old World primates (monkeys of Asia and Africa and apes) and humans, however. Anti-gal antibodies are produced in humans and primates as a result of an immune response to α-gal epitope carbohydrate structures on gastrointestinal bacteria. U. Galili et al., Infect. Immun. 56: 1730 (1988); R. M. Hamadeh et al., J. Clin. Invest. 89: 1223 (1992).

Since non-primate mammals (e.g., pigs) produce α-gal epitopes, xenotransplantation of ATM from these mammals into primates often results in immunological activation because of primate anti-Gal antibodies binding to these epitopes on the ATM. U. Galili et al., Immunology Today 14: 480 (1993); M. Sandrin et al., Proc. Natl. Acad. Sci. USA 90: 11391 (1993); H. Good et al., Transplant. Proc. 24: 559 (1992); B. H. Collins et al., J. Immunol. 154: 5500 (1995). Furthermore, xenotransplantation results in major activation of the immune system to produce increased amounts of high affinity anti-gal antibodies. Accordingly, in some embodiments, when animals that produce α-gal epitopes are used as the tissue source, the substantial elimination of α-gal epitopes from cells and from extracellular components of the ATM, and the prevention of re-expression of cellular α-gal epitopes can diminish the immune response against the ATM associated with anti-gal antibody binding to α-gal epitopes.

To remove α-gal epitopes, after washing the tissue thoroughly with saline to remove the DNase solution, the tissue sample may be subjected to one or more enzymatic treatments to remove certain immunogenic antigens, if present in the sample. In some embodiments, the tissue sample may be treated with an α-galactosidase enzyme to eliminate α-gal epitopes if present in the tissue. In some embodiments, the tissue sample is treated with α-galactosidase at a concentration of 300 U/L prepared in 100 mM phosphate buffer at pH 6.0. In other embodiments, the concentration of α-galactosidase is increased to 400 U/L for adequate removal of the a-gal epitopes from the harvested tissue. Any suitable enzyme concentration and buffer can be used as long as sufficient removal of antigens is achieved.

Alternatively, rather than treating the tissue with enzymes, animals that have been genetically modified to lack one or more antigenic epitopes may be selected as the tissue source. For example, animals (e.g., pigs) that have been genetically engineered to lack the terminal a-galactose moiety can be selected as the tissue source. For descriptions of appropriate animals see co-pending U.S. Application Serial No. 10/896,594 and U.S. Patent No. 6,166,288. In addition, certain exemplary methods of processing tissues to produce acellular matrices with or without reduced amounts of or lacking alpha-1,3-galactose moieties, are described in Xu.

After the ATM is formed, histocompatible, viable cells may optionally be seeded in the ATM to produce a graft that may be further remodeled by the host. In some embodiments, histocompatible viable cells may be added to the matrices by standard in vitro cell co-culturing techniques prior to transplantation, or by in vivo repopulation following transplantation. In vivo repopulation can be by the recipient's own cells migrating into the ATM or by infusing or injecting cells obtained from the recipient or histocompatible cells from another donor into the ATM in situ. Various cell types can be used, including embryonic stem cells, adult stem cells (e.g., mesenchymal stem cells), and/or neuronal cells. In various embodiments, the cells can be directly applied to the inner portion of the ATM just before or after implantation. In certain embodiments, the cells can be placed within the ATM to be implanted, and cultured prior to implantation. In some embodiments, viable cells may be added to the tissue scaffold at the desired anatomic site after the solvent has diffused from the scaffold.

In certain embodiments, the ATM can include ALLODERM® or STRATTICE™, LifeCell Corporation, Branchburg, NJ, which are human and porcine acellular dermal matrices respectively. Examples of such materials may be found in U.S. Patent Nos. 6,933,326 and 7,358,284.

### Particulate Acellular Tissue Matrix

The following procedure can be used to produce particulate acellular tissue matrices using ALLODERM®, STRATTICE™, or other suitable acellular tissue matrices. After removal from the packaging, ATM can be cut into strips using a Zimmer mesher fitted with a non-interrupting "continuous" cutting wheel. The resulting long strips of ATM may be cut into lengths of about 1 to about 2 centimeters in length.

A homogenizer and sterilized homogenizer probe, such as a LabTeck Macro homogenizer available from OMNI International, Warrenton Va., may be assembled and cooled to cryogenic temperatures using sterile liquid nitrogen which is poured into the homogenizer tower. Once the homogenizer has reached cryogenic temperatures, ATM previously prepared into strips as noted above can be added to the homogenizing tower containing sterile liquid nitrogen. The homogenizer may then be activated so as to cryogenically fracture the strips of ATM. The time and duration of the cryogenic fractionation step will depend upon the homogenizer utilized, the size of the homogenizing chamber, the speed and time at which the homogenizer is operated, and should be able to be determined by one of skill in the art by simple variation of the parameters to achieve the desired results.

The cryofractured particulate ATM material may be sorted by particle size by washing the product of the homogenizer with liquid nitrogen through a series of metal screens that have also been cooled to liquid nitrogen temperatures. A combination of screens may be utilized within the homogenizing tower of the type described above in which the particles are washed and sorted first to exclude oversized particles and then to exclude undersized particles.

Once isolated, the particulate ATM may be removed and placed in a vial for freeze drying once the sterile liquid nitrogen has evaporated. This may ensure that any residual moisture that may have been absorbed during the above procedure is removed.

The final product can be a powder having a particle size of about 1 micron to about 900 microns or a particle size of about 30 microns to about 750 microns. The particles are distributed about a mean of about 150-300 microns. The material is readily rehydrated by suspension in normal saline or other similar suitable rehydrating agent. The rehydrated ATM may be resuspended in normal saline or any other suitable pharmaceutically compatible carrier.

In certain embodiments, the particulate ATM can include CYMETRA®, LifeCell Corporation, Branchburg, NJ, which is an injectable form of ALLODERM®. Examples of such a material may be found in U.S. Patent Nos. 7,358,284 and 6,933,326.

The following examples are provided to better explain the various embodiments and should not be interpreted in any way to limit the scope of the present disclosure.

### Examples:

### Example 1

The effect of implantation of regenerative tissue scaffold with porcine dermal tissue derived particulate ATM (pADM) was compared to implantation of preformed polymer alone according to the procedures described below.

Regenerative tissue scaffolds were created from particulate ATM. ATM was prepared from porcine dermal tissue and was freeze dried. Dry ATM was cut into ∼1cm² pieces and placed into a cryomill vial. The vial was then placed in a SPEX 6800 freezer mill that had been pre-cooled with liquid nitrogen and subjected to a cryofracture protocol. The particulate ATM was then removed from the vial and maintained under dry storage conditions.

PCL combined with pADM and P4HB combined with pADM were each solubilized in each of dioxane and NMP according to the following procedure. The selected polymer was solubilized in the selected solvent at a concentration of 10% (w/v). 0.5 ml of this solution was then drawn into a 1 ml syringe. 150 mg of pADM was added to a 3 ml syringe. A connector was placed onto the 1 ml syringe, and all of the air was removed from the barrel. The plunger on the 3 ml syringe was pulled back to the 2 ml mark, and the syringe was tapped to loosen the pADM powder. The 3 ml syringe was then connected to the 1 ml syringe. The solution from the 1 ml syringe was slowly injected into the 3 ml syringe, allowing the pADM to become wetted. The 3 ml syringe was tapped repeatedly until the pADM appeared fully wet. The material was transferred between the 1 ml and 3 ml syringes approximately 10 times to evenly mix the polymer solution and the pADM, and the material was left in the 3 ml syringe when finished. The 1 ml syringe was disconnected and the plunger on the 3 ml syringe was retracted. The 3 ml syringe was tapped to pack the contents against the plunger. The plunger was slowly depressed, expelling all the air from the 3 ml syringe. The 1 ml syringe was then reconnected, and the polymer-pADM mixture was transferred repeatedly back and forth between the syringes for approximately 2 minutes. The polymer-pADM mixture was transferred to the 1 ml syringe, and a needle/cannula was attached for delivery.

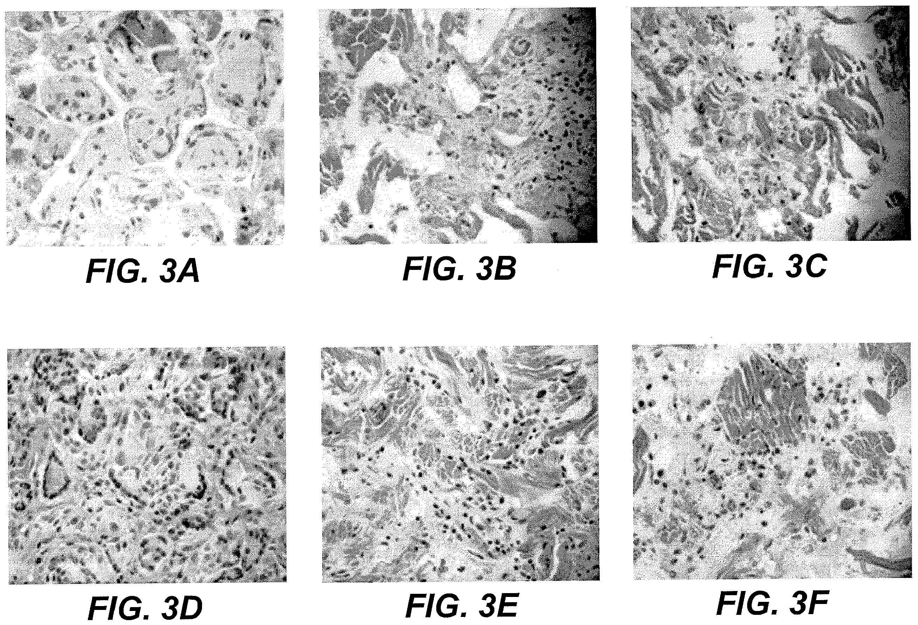

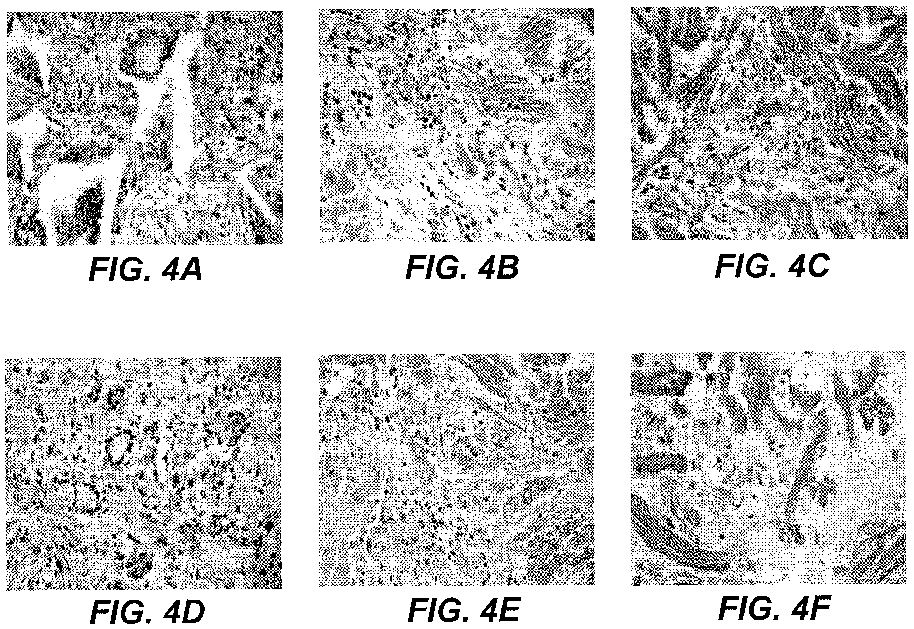

The various polymer-pADM mixtures described above, constructs comprising only PCL, and constructs comprising only P4HB were each implanted in a sub-dermal position through a small incision on the dorsal surface of immune-competent rats (*Rattus norvegicus*; Lewis Rat). Four weeks (Figs. 3A-F) and twelve weeks (Figs. 4A-F) after implantation, explants were collected and washed with PBS and were fixated in 10% formalin. Fixed tissue was embedded in paraffin and sections of tissue matrix samples were stained with hematoxylin and eosin (H&E) using standard procedures. D.C. Sheehan and B.B. Hrapchak, Theory and Practice of Histotechnology, 2nd edn., Columbus, OH, Battelle Press (1987).

Samples were then observed under a microscope at 400x magnification (Figs. 3A-F and 4A-F). Figures 3A-C depict the results of PCL alone, with pADM and dioxane, and with pADM and NMP, respectively, after 4 weeks. Figures 3D-F depict the results of P4HB alone, with pADM and dioxane, and with pADM and NMP, respectively, after 4 weeks. Similarly, Figures 4A-C depict the results of PCL alone, with pADM and dioxane, and with pADM and NMP, respectively, after 12 weeks. And figures 4D-F depict the results the P4HB alone, with pADM and dioxane, and with pADM and NMP, respectively, after 12 weeks. Histological analysis of the explants showed that PCL and P4HB in the presence of pADM, when solubilized in either of dioxane or NMP, had an attenuated inflammatory response compared to explants of PCL and P4HB alone.

### Example 2

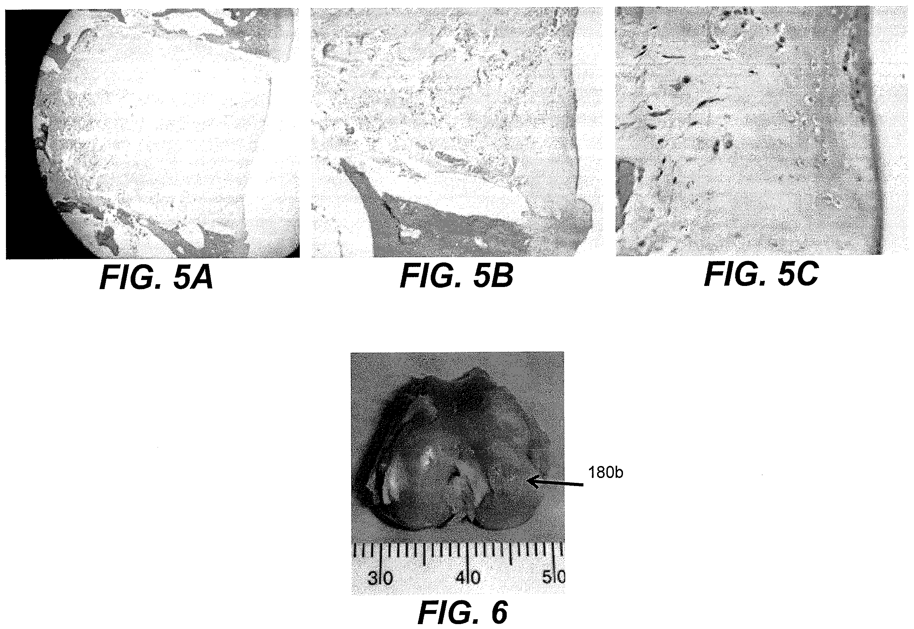

Implantation of a regenerative tissue scaffold in the femoral condyle of a rabbit was evaluated. An implant comprising BHA, pADM, and NMP was prepared according to the procedure described in Example 1. BHA was mixed with pADM in an NMP solvent and injected into an approximately 3.5 x 3 mm osteochondral defect created on the femoral condyle of a rabbit. Samples were removed following 4 weeks, and cellular responses were evaluated using routine histological staining with hematoxylin and eosin. The samples were observed under microscope at 20x, 100x, and 400x magnification (Figs. 5a-c, respectively). A photo of the femoral condyle showing the tissue scaffold implant 180b was also taken at 12 weeks (Fig. 6). The results showed the persistence of the implant and a regenerative tissue response as demonstrated by the deposition of hyaline-like cartilage at the site of the defect.

## Claims

1. A tissue scaffold mixture comprising:
a particulate acellular tissue matrix (ATM); a water miscible solvent and a polymer dissolved in the solvent;
wherein the water miscible solvent is capable of diffusing into aqueous media placed in contact with the mixture, so as to allow the polymer and ATM to form a tissue scaffold.

2. The tissue scaffold mixture of claim 1, wherein the tissue scaffold has a stable three-dimensional shape.

3. The tissue scaffold mixture of any preceding claim, wherein the polymer comprises polycaprolactone.

4. The tissue scaffold mixture of any one of claims 1 or 2, wherein the polymer comprises poly-4-hydroxybutyrate.

5. The tissue scaffold mixture of any one of claims 1 or 2, wherein the polymer comprises a benzyl ester derivative of hyaluronic acid.

6. The tissue scaffold mixture of any preceding claim, wherein the particulate ATM comprises uniform size particles.

7. The tissue scaffold mixture of any preceding claim, wherein the particulate ATM comprises a dermal ATM, a cartilage tissue matrix, a bone tissue matrix, or combination thereof.

8. The tissue scaffold mixture of any preceding claim, wherein the particulate ATM comprises ATM from two or more different types of tissues.

9. The tissue scaffold mixture of claim 8, wherein the two or more different types of tissues comprise dermis and cartilage, cartilage and bone, human tissue matrices, porcine tissue matrices, or human tissue matrices and porcine tissue matrices.

10. The tissue scaffold mixture of any preceding claim, wherein the solvent is biocompatible.

11. The tissue scaffold mixture of any preceding claim, wherein the solvent comprises at least one of dioxane, N-methyl-2-pyrrolidone, or dimethyl sulfoxide, or combinations thereof.

12. A tissue scaffold mixture according to any preceding claim, for use in implantation in an animal, wherein the water miscible solvent is capable of diffusing from the mixture to allow the mixture to form a tissue scaffold from the polymer and ATM.

13. The tissue scaffold mixture of claim 12, for treating a tissue site.

14. The tissue scaffold mixture of claim 13, wherein the tissue is bone, cartilage, or breast.

15. The tissue scaffold mixture of claim 12 or 13, wherein placing the mixture in contact with an aqueous media comprises placing the mixture in, on, or proximate to a tissue site.

16. The tissue scaffold mixture of claim 15, wherein placing the mixture in contact with an aqueous media comprises injecting the mixture into the tissue site, onto the tissue site, or a combination thereof.

## Patentansprüche

1. Gewebegerüstgemisch, umfassend:
eine partikelförmige azelluläre Gewebematrix (ATM); ein wassermischbares Lösungsmittel und ein in dem Lösungsmittel gelöstes Polymer;
worin das wassermischbare Lösungsmittel zum Diffundieren in wässrige, mit dem Gemisch in Kontakt gebrachte Medien fähig ist, damit das Polymer und die ATM ein Gewebegerüst bilden können.

2. Gewebegerüstgemisch nach Anspruch 1, worin das Gewebegerüst eine stabile dreidimensionale Form aufweist.

3. Gewebegerüstgemisch nach einem vorhergehenden Anspruch, worin das Polymer Polycaprolacton umfasst.

4. Gewebegerüstgemisch nach einem der Ansprüche 1 bis 3, worin das Polymer Poly-4-hydroxybutyrat umfasst.

5. Gewebegerüstgemisch nach einem der Ansprüche 1 bis 3, worin das Polymer ein Benzylesterderivat von Hyaluronsäure umfasst.

6. Gewebegerüstgemisch nach einem vorhergehenden Anspruch, worin die partikelförmige ATM gleichmäßig große Partikel umfasst.

7. Gewebegerüstgemisch nach einem vorhergehenden Anspruch, worin die partikelförmige ATM eine dermale ATM, eine Knorpelgewebematrix, eine Knochengewebematrix oder Kombination davon umfasst.

8. Gewebegerüstgemisch nach einem vorhergehenden Anspruch, worin die partikelförmige ATM eine ATM aus zwei oder mehr verschiedenen Gewebetypen umfasst.

9. Gewebegerüstgemisch nach Anspruch 8, worin die zwei oder mehr verschiedenen Gewebetypen Dermis und Knorpel, Knorpel und Knochen, humane Gewebematrizen, porkine Gewebematrizen oder humane Gewebematrizen und porkine Gewebematrizen umfassen.

10. Gewebegerüstgemisch nach einem vorhergehenden Anspruch, worin das Lösungsmittel biokompatibel ist.

11. Gewebegerüstgemisch nach einem vorhergehenden Anspruch, worin das Lösungsmittel mindestens eines von Dioxan, N-Methyl-2-pyrrolidon oder Dimethylsulfoxid oder Kombinationen davon umfasst.

12. Gewebegerüstgemisch nach einem vorhergehenden Anspruch zur Verwendung bei der Implantation in einem Tier, worin das wassermischbare Lösungsmittel zum Diffundieren aus dem Gemisch fähig ist, damit das Gemisch ein Gewebegerüst aus dem Polymer und der ATM bilden kann.

13. Gewebegerüstgemisch nach Anspruch 12, zur Behandlung einer Gewebestelle.

14. Gewebegerüstgemisch nach Anspruch 13, worin das Gewebe Knochen, Knorpel oder Brust ist.

15. Gewebegerüstgemisch nach Anspruch 13 oder 14, worin das Inkontaktbringen des Gemisches mit einem wässrigen Medium das Platzieren des Gemisches in, auf oder nahe einer Gewebestelle umfasst.

16. Gewebegerüstgemisch nach Anspruch 15, worin Inkontaktbringen des Gemisches mit einem wässrigen Medium das Injizieren des Gemisches in die Gewebestelle, auf die Gewebestelle oder eine Kombination davon umfasst.

## Revendications

1. Mélange d'échafaudages tissulaires comprenant :
une matrice de tissu acellulaire (ATM) particulaire ; un solvant miscible dans l'eau et un polymère dissout dans le solvant ;
le solvant miscible dans l'eau étant capable de se diffuser dans un milieu aqueux placé en contact avec le mélange, de manière à permettre au polymère et à l'ATM de former un échafaudage tissulaire.

2. Mélange d'échafaudages tissulaires selon la revendication 1, dans lequel l'échafaudage tissulaire a une forme tridimensionnelle stable.

3. Mélange d'échafaudages tissulaires selon l'une quelconque des revendications précédentes, dans lequel le polymère comprend de la polycaprolactone.

4. Mélange d'échafaudages tissulaires selon l'une quelconque des revendications 1 à 3, dans lequel le polymère comprend du poly-4-hydroxybutyrate.

5. Mélange d'échafaudages tissulaires selon l'une quelconque des revendications 1 à 3, dans lequel le polymère comprend un dérivé d'ester de benzyle d'acide hyaluronique.

6. Mélange d'échafaudages tissulaires selon l'une quelconque des revendications précédentes, dans lequel l'ATM particulaire comprend des particules de taille uniforme.

7. Mélange d'échafaudages tissulaires selon l'une quelconque des revendications précédentes, dans lequel l'ATM particulaire comprend une ATM dermique, une matrice de tissu cartilagineux, une matrice de tissu osseux ou une combinaison correspondante.

8. Mélange d'échafaudages tissulaires selon l'une quelconque des revendications précédentes, dans lequel l'ATM particulaire comprend une ATM d'au moins deux types différents de tissus.

9. Mélange d'échafaudages tissulaires selon la revendication 8, dans lequel les au moins deux types différents de tissus comprennent des matrices dermiques et cartilagineuses, des matrices cartilagineuses et osseuses, des matrices de tissus humains, des matrices de tissus porcins ou des matrices de tissus humains et des matrices de tissus porcins.

10. Mélange d'échafaudages tissulaires selon l'une quelconque des revendications précédentes, dans lequel le solvant est biocompatible.

11. Mélange d'échafaudages tissulaires selon l'une quelconque des revendications précédentes, dans lequel le solvant comprend au moins un dioxane, du N-méthyl-2-pyrrolidone, du diméthyl sulfoxide ou des combinaisons correspondantes.

12. Mélange d'échafaudages tissulaires selon l'une quelconque des revendications précédentes, destiné à être utilisé dans une implantation chez un animal, le solvant miscible dans l'eau étant capable de se diffuser du mélange pour permettre au mélange de former un échafaudage tissulaire à partir du polymère et de l'ATM.

13. Mélange d'échafaudages tissulaires selon la revendication 12, destiné à traiter un site tissulaire.

14. Mélange d'échafaudages tissulaires selon la revendication 13, dans lequel le tissu est un os, un cartilage ou un sein.

15. Mélange d'échafaudages tissulaires selon la revendication 13 ou 14, dans lequel la mise en contact du mélange avec un milieu aqueux consiste à placer le mélange dans un site tissulaire ou à proximité d'un site tissulaire.

16. Mélange d'échafaudages tissulaires selon la revendication 15, dans lequel la mise en contact du mélange avec un milieu aqueux consiste à injecter le mélange dans le site tissulaire et/ou sur le site tissulaire.
